# FASCICULE DE BREVET EUROPEEN

(11) **EP 0 425 359 B1**
(45) Date de publication et mention de la délivrance du brevet: **11.10.1995**
(21) Numéro de dépôt: 90402967.5
(22) Date de dépôt: 22.10.1990
(51) Int. Cl.: C07D 307/80

(54) **Procédé de préparation de dérivés de benzoyl-3 benzofuranne**
Verfahren zur Herstellung von 3-Benzoylbenzofuranderivaten
Process for the preparation of 3-benzoyl-benzofuran derivatives

(30) Priorité: 23.10.1989 FR 8913851
(43) Date de publication de la demande: 02.05.1991
(73) Titulaire: SANOFI, 75008 Paris (FR)
(72) Inventeur: Boudet, Bernard, F-04200 Sisteron (FR); Dormoy, Jean-Robert, F-04200 Sisteron (FR); Heymes, Alain, F-04200 Sisteron (FR)
(74) Mandataire: Polus, Camille

(56) Documents cités:
- EP-A- 0 210 156
- CHEMICAL ABSTRACTS, vol. 94, no. 23, 8 juin 1981, page 640, résumé no. 192122b, Columbus, Ohio, US; && HU-A-18 236 (E. GY. T. GYOGYSZERVEGYESZETI GYAR) 28-05-1980
- JOURNAL OF ORGANIC CHEMISTRY, vol. 48, no. 19, 23 septembre 1983, pages 3214-3219, American Chemical Society, Easton, US; M. KAKUSHIMA et al.: "Regioselective synthesis of acylpyrroles"

## Description

La présente invention se rapporte, d'une manière générale, à un nouveau procédé de préparation de dérivés de benzoyl-benzofuranne.

Plus précisément, l'invention a pour objet un nouveau procédé de préparation de dérivés de benzoyl-3 benzofuranne de formule générale : 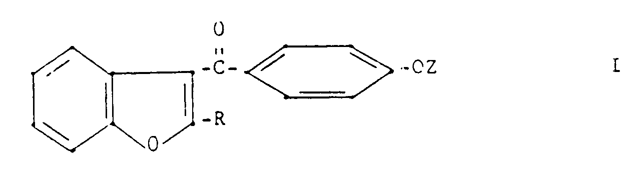
dans laquelle
R est choisi parmi un radical alkyle linéaire ou ramifié en C₁-C₈, un radical cycloalkyle en C₃-C₆ et un groupe phényle non substitué ou substitué par un ou plusieurs substituants identiques ou différents, choisis parmi des atomes d'halogène, par exemple fluor, chlore ou brome et des groupes alkyle en C₁-C₄, alkoxy en C₁-C₄ et nitro,
Z est choisi parmi l'hydrogène et le radical méthyle.

Par "radical alkyle linéaire ou ramifié en C₁-C₈", on désigne notamment les radicaux méthyle, éthyle, n-propyle, isopropyle, n-butyle, isobutyle, tertiobutyle, n-pentyle, néopentyle, n-hexyle, n-heptyle ou n-octyle.

De même, par "radical cycloalkyle en C₃-C₆", on entend notamment les radicaux cyclopropyle ou cyclohexyle.

Ainsi, en tenant compte des valeurs données ci-dessus, R peut représenter notamment un radical méthyle, éthyle, n-propyle, isopropyle, n-butyle, isobutyle, tertiobutyle, méthyl-1 propyle, n-pentyle, néopentyle, phényle, mono-fluoro-, mono-chloro- ou mono-bromo-phényle, difluoro-, dichloro- ou dibromo-phényle, mono-méthyl- ou di-méthyl- phényle, mono-méthoxy- ou di-méthoxy-phényle ou un radical méthyl-phényle substitué par un atome d'halogène.

Par "radical alkyle en C₁-C₄", on entend plus particulièrement, un radical méthyle, éthyle, n-propyle, isopropyle, n-butyle, isobutyle ou tertiobutyle.

Par "radical alkoxy en C₁-C₄", on désigne un groupement hydroxyle substitué par un radical alkyle en C₁-C₄ tel qu'explicité ci-dessus.

Parmi les composés de formule I, ceux dans laquelle R représente un radical éthyle ou n-butyle constituent des composés préférés.

Les dérivés de benzoyl-3 benzofuranne de formule I peuvent être largement utilisés comme produits intermédiaires notamment pour la synthèse finale de dérivés de benzofuranne décrits dans les brevets français n° 1 260 578 et 1 339 389.

De tels dérivés sont notamment la benziodarone ou éthyl-2 (diiodo-3,5 hydroxy-4 benzoyl)-3 benzofuranne, la benzbromarone ou éthyl-2 (dibromo-3,5 hydroxy-4 benzoyl)-3 benzofuranne et l'amiodarone ou n-butyl-2 (diiodo-3,5β-diéthylaminoéthoxy-4 benzoyl)-3 benzofuranne.

Ces composés se sont révélés particulièrement intéressants pour leurs applications thérapeutiques.

Ainsi, la benziodarone s'est montrée utile par son action coronarodilatatrice et hypouricémiante, la benzbromarone par son effet hypouricémiant et l'amiodarone par ses propriétés antiangineuses et antiarythmiques cardiaques.

Certains dérivés de formule I se sont également révélés utiles comme agents thérapeutiques. On peut citer par exemple l'éthyl-2 (hydroxy-4 benzoyl)-3 benzofuranne ou benzarone qui s'est montrée efficace par son effet phlébotonique et freinateur des réactions inflammatoires capillaroveineuses.

La préparation de dérivés d'anisoyl-3 ou benzoyl-3 benzofuranne, par réaction de Friedel-Crafts, est largement connue dans la littérature chimique.

A titre d'exemple, on peut citer :
- le brevet français n° 1 260 578 qui fait état de la réaction entre un alkyl-2 benzofuranne et le chlorure d'anisoyle en présence de chlorure stannique comme catalyseur et dans le sulfure de carbone comme solvant.

Le chlorure d'anisoyle peut être obteu par exemple à partir d'acide anisique et de chlorure de thionyle.
- Eur. J. Med. Chem. -Chimica Therapeutica 1974, 9, N° 1, pp. 19-25 qui décrit la condensation d'un dérivé alkyl-2 chlorocarbonyl-3 bennzofuranne avec un dialkyl-3,5 anisole en présence de chlorure d'aluminium comme catalyseur et dans le dichloréthane comme solvant, le dérivé chlorocarbonyl-3 étant obtenu selon la suite d'étapes suivantes :
   a) réaction d'un alkyl-2 benzofuranne avec le chlorure d'acétyle en présence de chlorure stannique,
   b) réaction de l'alkyl-2 acétyl-3 benzofuranne obtenu, avec le brome ou le chlorure de sulfuryle,
   c) chauffage, dans la pyridine, de l'alkyl-2 haloacétyl-3 benzofuranne obtenu,
   d) hydrolyse, en présence d'hydroxyde de sodium, de l'alkyl-2 ω - halopyridylium-acétyl-3 benzofuranne obtenu,
   e) régénération de l'acide alkyl-2 benzofuranne carboxylique-3 à partir de son sel sodique ainsi obtenu,
   f) réaction de l'acide alkyl-2 benzofuranne carboxylique-3 avec le chlorure de thionyle pour fournir l'alkyl-2 chlorocarbonyl-3 benzofuranne.

D'autres catalyseurs de Lewis ont été décrits dans la réaction de Friedel-Crafts parmi lesquels on peut citer, par exemple, le chlorure ferrique (demande de brevet EP-A-0 210 156) ou le chlorure d'aluminium.

Ce dernier a été utilisé dans un procédé rapporté dans le brevet hongrois n° 175 906 (Chem. Abstr. 94, 192122b) selon lequel, on prépare l'éthyl-2 (hydroxy-4 benzoyl)-3 benzofuranne de la manière suivante :
a) on effectue la réaction d'un équivalent d'ethyle-2 benzofuranne avec un équivalent de chlorure d'anisoyle (préparé extemporanément à partir d'acide anisique et de chlorure de thionyle) en présence de 1,02 équivalent de chlorure d'aluminium dans le chlorobenzène à 0°C,
b) on déméthyle le complexe obtenu par ajout de 3,08 équivalents de chlorure d'aluminium et on chauffe à 70°C,
c) on hydrolyse, à température ambiante pour obtenir, après purification et séparation, le composé désiré avec un rendement de 50,5%.

En conclusion, les méthodes évoquées ci-dessus font toutes appel à un procédé mettant en oeuvre pour la formation de la cétone finale, une seule réaction de Friedel-Crafts, le substituant chlorocarbonyle nécessaire à la condensation étant fixé au départ soit sur le dérivé de benzofuranne soit sur le dérivé de méthoxybenzène.

En conséquence, ces procédés antérieurs nécessitent la préparation intermédiaire du dérivé chlorocarbonyle approprié, ce qui peut, dans certains cas, augmenter considérablement le nombre total d'étapes du procédé et, corollairement, le prix de revient du produit final.

La recherche d'un procédé industriel mettant en oeuvre des intermédiaires de synthèse d'accès facile et peu onéreux ainsi qu'un nombre restreint d'étapes et un rendement satisfaisant en produit final reste, par conséquent, d'un intérêt incontestable.

On sait, par ailleurs, que la réaction de Friedel-Crafts peut être accomplie également à partir du phosgène ou du chlorure d'oxalyle sur un substrat aromatique Ar.

Dans ce cas, on obtient la cétone symétrique Ar-CO-Ar comme produit majoritaire accompagnée d'une très faible quantité d'acide ArCO₂H selon le schéma : 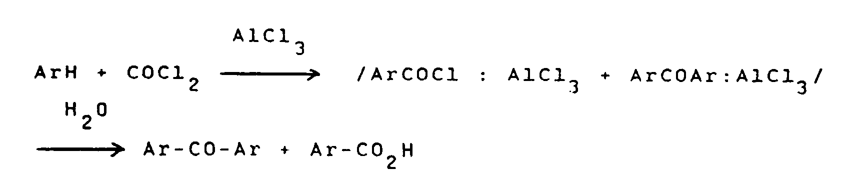
La seule méthode pour obtenir l'acide avec un bon rendement consiste à utiliser le sulfure de carbone comme solvant car le complexe /ArCOCl : AlCl₃/ n'y étant pas soluble, précipite et ne peut donc réagir avec une deuxième molécule d'ArH pour donner la cétone symétrique.

L'obtention de cétones symétriques dans ce type de réaction limite, par conséquent, la portée d'un procédé de préparation de cétones par double réaction de Friedel-Crafts entre deux composés aromatiques différents et le phosgène.

Dans l'état actuel des connaissances, il n'existerait pas d'exemple de préparation de cétones aromatiques non symétriques basé sur ce type de procédé.

Or, on a maintenant trouvé selon l'invention, qu'il est possible d'obtenir des cétones aromatiques non symétriques en l'occurence les dérivés de benzoyl-3 benzofuranne de formule I par double réaction de Friedel-Crafts mettant en oeuvre le phosgène et deux composés aromatiques différents.

Ainsi, selon l'invention, on prépare les composés de formule I en faisant réagir in situ en présence de chlorure d'aluminium comme catalyseur,à une température comprise entre -25°C et la température ambiante et dans un solvant aprotique apolaire, un dérivé de benzofuranne de formule générale : 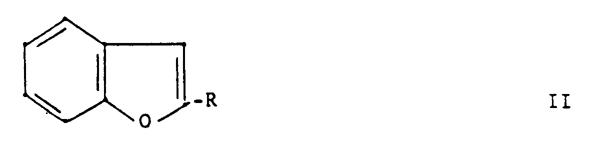
dans laquelle R a la même signification que précédemment, d'abord avec le phosgène ou le chlorure d'oxalyle ensuite avec un dérivé phénolique de formule générale : 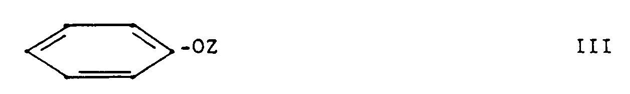
dans laquelle Z à la même signification que précédemment, pour obtenir un complexe que l'on hydrolyse pour former le composé désiré de formule I.

On a remarqué que les proportions relatives des divers réactifs en présence jouent un rôle non négligeable sur la formation de produits secondaires représentés par des cétones symétriques.

Les rapports molaires les plus convenables utilisés dans le procédé de l'invention en vue de favoriser la production de cétones non symétriques se sont révélés comme suit :
composé de formule II ou III/phosgène ou chlorure d'oxalyle/chlorure d'aluminium: 1 / 2 à 4 / 1 à 1,5 avec un préférence pour le rapport 1 / 3 / 1,5, les composés de formule II et III étant en quantité stoéchiométrique.

Le solvant aprotique apolaire utilisé dans le procédé de l'invention est, de préférence, le tétrachlorure de carbone ou un halogénure d'alkyle en C₁-C₄ tel que le dichlorométhane ou le dichloroéthane.

Quant à l'hydrolyse, celle-ci a lieu à une température comprise entre la température ambiante et 50°C, de préférence à la température ambiante.

Le procédé de l'invention peut être mis en oeuvre en chargeant dans un réacteur à une température de l'ordre de -20°C, le dérivé de benzofuranne de formule II, le phosgène, le catalyseur et le solvant choisi, et en effectuant ensuite la réaction à température ambiante pendant plusieurs heures, par exemple de 20 à 24 heures.

On introduit alors le dérivé de formule III dans le milieu réactionnel à une température de l'ordre de -20°C, puis on réchauffe pour effectuer la réaction à température ambiante pendant 2 à 3 heures et on verse le milieu réactionnel dans l'eau pour réaliser l'hydrolyse du complexe.

Le dérivé de benzoyl-3 benzofuranne de formule I ainsi obtenu peut être ensuite séparé de manière classique.

Le procédé de l'invention, ainsi décrit permet d'obtenir les dérivés de benzoyl-3 benzofuranne de formule I avec facilité et excellents rendements. Par exemple, le n-butyl-2 (méthoxy-4 benzoyl)-3 benzofuranne peut être produit, selon l'invention, avec des rendements supérieurs à 75%.

En outre, le procédé de l'invention présente, sur les procédés antérieurs, l'avantage incontestable, d'éviter la préparation extemporanée d'un dérivé chlorocarbonyle et d'ainsi réduire considérablement le nombre d'étapes.

On peut également préparer un dérivé de formule I dans laquelle Z représente l'hydrogène à partir du dérivé de (méthoxy-4 benzoyl)-3 benzofuranne de formule I que l'on déméthyle en dérivé d'(hydroxy-4 benzoyl)-3 benzofuranne par exemple par application de la méthode décrite dans le brevet EP-A-0 210 156, c'est-à-dire en faisant intervenir le chlorure d'aluminium comme agent de déméthylation à la température de reflux du milieu réactionnel.

Les dérivés de benzoyl-3 benzofuranne de formule I dans laquelle Z représente l'hydrogène peuvent, en outre, être préparés à partir d'un composé de formule II dans laquelle Z représente le groupe méthyle suivie d'une déméthylation in situ du complexe formé au moyen de chlorure d'aluminium, le milieu réactionnel étant à la température de reflux.

Ainsi, selon cette variante du procédé, on fait réagir in situ en présence de chlorure d'aluminium comme catalyseur à une température comprise entre -25°C et la température ambiante et dans un solvant aprotique apolaire, un dérivé de benzofuranne de formule II, d'abord avec le phosgène ou le chlorure d'oxalyle, ensuite avec l'anisole pour obtenir un complexe que l'on déméthyle in situ à la température de reflux du milieu et en présence de chlorure d'aluminium puis on effectue une hydrolyse pour obtenir les dérivés de benzoyl-3 benzofuranne de formule I dans laquelle Z représente l'hydrogène.

La quantité de chlorure d'aluminium nécessaire aux réactions de Friedel-Crafts est comme indiqué précédemment de 1 à 1,5 équivalent par équivalent de composé de formule II tandis que la quantité de chlorure d'aluminium nécessaire à la déméthylation est d'un équivalent par équivalent de composé de formule II.

Cette variante du procédé présente notamment l'avantage de pouvoir réaliser l'ensemble des réactions nécessaires à l'obtention des dérivés (hydroxy-4 benzoyl)-3 benzofuranne de formule I dans un seul milieu réactionnel, sans aucun isolement de produit intermédiaire.

En outre, puisque l'on utilise le chlorure d'aluminium à la fois pour les réactions de Friedel-Crafts et pour la déméthylation, on peut diminuer de manière très sensible la quantité de cet agent par rapport aux quantités préconisées dans le brevet hongrois n° 175.906 qui prévoit l'utilisation de 4 équivalents de chlorure d'aluminium au total.

La variante de procédé ainsi décrite permet de préparer les dérivés d'(hydroxy-4 benzoyl)-3 benzofuranne de formule I avec de très bons rendements. Dans le cas notamment de la préparation du n-butyl-2 (hydroxy-4 benzoyl)-3 benzofuranne, on a pu, en appliquant cette variante du procédé, obtenir des rendements d'au moins 70%.

Comme indiqué précédemment, les dérivés de benzoyl-3 benzofuranne de formule I peuvent être utilisés pour la préparation de la benziodarone, de la benzbromarone ou de l'amiodarone.

Par exemple, la benziodarone et la benzbromarone peuvent être préparées, notamment, par iodation ou bromation respective de l'éthyl-2 (hydroxy-4 benzoyl)-3 benzofuranne en phase homogène et en présence d'une solution tampon acétate de métal alcalin/acide acétique et l'amiodarone peut être obtenue par iodation du n-butyl-2 (hydroxy-4 benzoyl)-3 benzofuranne, de manière analogue à celle décrite précédemment, pour obtenir le n-butyl-2 (diiodo-3,5 hydroxy-4 benzoyl)-3 benzofuranne suivie d'une éthérification par le chlorhydrate de diéthylamino-1 chloro-2 éthane en présence d'une solution tampon carbonate de métal alcalin/bicarbonate de métal alcalin.

Les exemples suivants illustrent l'invention et la préparation de l'amiodarone.

### EXEMPLE 1

### Préparation du n-butyl-2 (méthoxy-4 benzoyl)-3 benzofuranne

On place dans un réacteur, sous argon, 40 ml de dichloréthane anhydre et on refroidit à -20°C. On introduit alors successivement 3 ml (41,87 mmoles) de phosgène, 2,5 ml (14,2 mmoles) de n-butyl-2 benzofuranne et 2,13 g (15,96 mmoles) de chlorure d'aluminium dans le réacteur. On réchauffe la solution progressivement à température ambiante, agite durant 24 heures et refroidit à -20°C.

On ajoute alors 1,55 ml (14,25 mmoles) d'anisole puis on réchauffe la solution à la température ambiante, agite pendant 2 heures et verse dans 100 ml d'eau. Après 30 mn sous agitation vive, on sépare la phase organique, lave avec une solution saturée de 30 ml de bicarbonate de sodium avec 40 ml d'eau puis on sèche sur sulfate de sodium.

Après évaporation du solvant et purification du produit brut obtenu sur une colonne de silice (éluant : chlorure de méthylène/hexane 1/1), on obtient 3,44 g (11,17 mmoles) de n-butyl-2 (méthoxy-4 benzoyl)-3 benzofuranne.
Rendement : 78,7 %.

### EXEMPLE 2

### Préparation du n-butyl-2 (hydroxy-4 benzoyl)-3 benzofuranne

Dans un ballon sec, on place, sous argon, 40 ml de dichloréthane anhydre et on refroidit à -20°C. On introduit alors successivement dans le ballon 3 ml (41,87 mmoles) de phosgène, 2,5 ml (14,2 mmoles) de n-butyl-2 benzofuranne et 2,13 g (15,96 mmoles) de chlorure d'aluminium.

On réchauffe la solution à température ambiante, agite durant 24 heures et refroidit à -20°C. On ajoute ensuite 1,55 ml (14,25 mmoles) d'anisole puis on réchauffe la solution à 25°C.

On agite pendant 2 heures et on ajoute 1,9 g (14,23 mmoles) de chlorure d'aluminium. On porte le mélange au reflux pendant 8 heures. Après retour à 40°C environ, on vers le milieu réactionnel dans 100 ml d'eau et on agite le mélange pendant 30 mn. On sépare alors la phase organique, lave avec 30 ml d'une solution saturée de bicarbonate de sodium puis avec 40 ml d'eau. On sèche sur sulfate de sodium et on effectue une décoloration. Après évaporation du solvant et purification du produit brut obtenu sur une colonne de silice (éluant : chlorure de méthylène), on obtient 2,9 g (9,87 mmoles) de n-butyl-2 (hydroxy-4 benzoyl)-3 benzofuranne.
Rendement : 70%
P.F. : 120°C.

### EXEMPLE 3

### Préparation du chlorhydrate de n-butyl-2 (diiodo-3,5 β-diéthylamino-éthoxy-4 benzoyl)-3 benzofuranne

### a) n-Butyl-2 (hydroxy-4 benzoyl)-3 benzofuranne

A une solution de 2 g (6,5 mmoles) de n-butyl-2 (méthoxy-4 benzoyl)-3 benzofuranne dans 30 ml de dichloréthane, on ajoute, sous argon, 1,82 g (13,6 mmoles) de chlorure d'aluminium. On chauffe le mélange durant 9 heures à reflux puis on refroidit à température ambiante. On verse alors le milieu réactionnel dans 50 ml d'eau, on agite ensemble vigoureusement durant 30 minutes puis on sépare la phase organique. On lave 3 fois avec 30 ml d'eau, sèche sur sulfate de sodium et décolore sur terre de diatomées. Après évaporation du solvant, on obtient 1,9 g (6,5 mmoles) de n-butyl-2 (hydroxy-4 benzoyl)-3 benzofuranne.
Rendement : 100%
P.F. : 119-120°C.

### b) n-Butyl-2 (diiodo-3,5 hydroxy-4 benzoyl)-3 benzofuranne

Dans un réacteur de 4l, on introduit 540 g de méthanol. Sous agitation, on ajoute alors successivement 300 g (2,4 moles) d'acétate de sodium trihydraté, 286 g d'iode et 424 g d'iode récupéré humide (2,3 moles au total). On amène le milieu réactionnel à 30 à 35°C et on introduit en une fois 294 g (1 mole) de n-butyl-2 (hydroxy-4 benzoyl)-3 benzofuranne.

On rince avec 60g de méthanol et on porte le mélange au reflux en 30 à 45 mn (température dans la masse : 70 à 74°C). On arrête le chauffage et on introduit, en 10 mn environ, une solution préalablement préparée de 90g (2,2 moles) d'hydroxyde de sodium en écailles dans 400 g d'eau épurée. L'exothermicité de la réaction maintient le reflux (76/77°C dans la masse) pendant le temps d'introduction. On maintient pendant 2 h à reflux puis on modifie l'appareil en un appareil de distillation à pression atmosphérique. On introduit, en 20 mn environ, une solution aqueuse de 320 g de bisulfite de sodium (35°Bé) et on poursuit la distillation jusqu'à atteindre 97 à 100°C dans la masse (température de tête : 87°C). On distille ainsi environ 800 ml (environ 680g) de solvant. On refroidit au moyen d'un bain d'eau à 75 à 80°C et on introduit successivement et dans l'ordre 200 g d'eau épurée, 215 g d'acide chlorhydrique à 36% et 1600 g de toluène. On porte au reflux pendant 10 mn (température de la masse : 84°C - dégagement d'anhydride sulfurique) et on décante la phase aqueuse inférieure. On lave la couche toluénique à 75 à 80°C successivement avec 400 g d'eau épurée, 100 g d'une solution aqueuse de bisulfite de sodium et 2 fractions chacune de 400 g d'eau épurée. On décante au maximum après le dernier lavage et on traite avec 22 g de charbon actif durant 30 mn au reflux. On filtre à chaud, on rince avec 380 g de toluène chaud et on réunit les filtrats.

On obtient ainsi une solution toluénique de n-butyl-2 (diiodo-3,5 hydroxy-4 benzoyl)-3 benzofuranne que l'on utilise telle quelle.

De la même manière que celle décrite précédemment mais à partir de 266,3 g (1 mole) d'éthyl-2 (hydroxy-4 benzoyl)-3 benzofuranne, on obtient l'éthyl-2 (diiodo-3,5 hydroxy-4 benzoyl)-3 benzofuranne ou benziodarone après élimination du toluène par distillation.

De même, à partir de 266,3 g (1 mole) d'ethyl-2 (hydroxy-4 benzoyl)-3 benzofuranne et de brome, on obtient l'éthyl-2 (dibromo-3,5 hydroxy-4 benzoyl)-3 benzofuranne ou benzbromarone après élimination du toluène par distillation.

### c) n-butyl-2 (diiodo-3,5 β-diéthylaminoéthoxy-4 benzoyl)-3 benzofuranne

Dans un réacteur, on introduit la solution toluénique de n-butyl-2 (diiodo-3,5 hydroxy-4 benzoyl)-3 benzofuranne obtenue au paragraphe b) ainsi que 800 g d'eau épurée et 177,3 g (1,03 mole) de chlorhydrate de diéthylamino-1 chloro-2 éthane. Sous agitation, on amène le milieu réactionnel à 40 ± 2°C et on l'y maintient durant 15 mn. En contrôlant le départ d'anhydride carbonique, on introduit alors lentement en pluie 416 g (3 moles) de carbonate de potassium anhydre. On élève progressivement la température pour atteindre le reflux en 1 h. On maintient durant 3 h à cette température, on décante la couche aqueuse saline à 75 ± 5°C et on lave la couche toluénique, à cette température, avec 4 fractions chacune de 800 g d'eau épurée. A la température de 60°C, on traite la solution toluénique avec 20,5 g de charbon actif, on filtre, on rince avec environ 220g de toluéne et on réunit les filtrats.

On obtient ainsi une solution toluénique de n-butyl-2 (diiodo-3,5 β-diéthylaminoéthoxy-4 benzoyl)-3 benzofuranne sous forme de base ou amiodarone.

### d) chlorhydrate de n-butyl-2 (diiodo-3,5 β-diéthylaminoéthoxy-4 benzoyl)-3 benzofuranne

On amène à 60°C la solution de n-butyl-2 (diiodo-3,5 β-diéthylaminoéthoxy-4 benzoyl)-3 benzofuranne obtenue au paragraphe c) et on introduit en 45 mn 38,5 g de gaz chlorhydrique au moyen d'un tube plongeant. On laisse la température de la masse s'élever suite à l'exothermicité de la réaction sans toutefois dépasser 75°C.

On vérifie que le pH est franchement acide en fin d'introduction et après 30 mn de contact à 70 ± 5°C.
On met progressivement sous vide de la trompe à l'eau et on distille environ 400 ml de toluène/eau/acide chlorhydrique (fin de distillation : pression résiduelle ≦ 150 mm; température de la masse ≦ 75°C. On cristallise sous agitation lente avec bain d'eau durant environ 8 heures et on essore à 10 à 15°C. On rince avec 4 fractions chacune de 180 ml de toluène filtré et on sèche en étuve ventilée à 60°C jusqu'à ce que le poids soit constant.

On obtient ainsi environ 647,5 g de chlorhydrate de n-butyl-2 (diiodo-3,5 β-diéthylaminoéthoxy-4 benzoyl)-3 benzofuranne ou chlorhydrate d'amiodarone.

Rendement : environ 95% (par rapport au n-butyl-2 (hydroxy-4 benzoyl)-3 benzofuranne).

## Revendications

1. Procédé de préparation de dérivés de benzoyl-3 benzofuranne de formule générale : 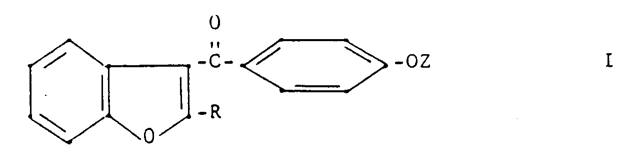 dans laquelle
R est choisi parmi un radical alkyle linéaire ou ramifié en C₁-C₈, un radical cycloalkyle en C₃-C₆ et un groupe phényle non substitué ou substitué par un ou plusieurs substituants identiques ou différents, choisis parmi des atomes d'halogène, des groupes alkyle en C₁-C₄, alkoxy en C₁-C₄ et nitro,
Z est choisi parmi l'hydrogène et le radical méthyle, caractérisé en ce que l'on fait réagir successivement in situ, en présence de chlorure d'aluminium comme catalyseur, à une température comprise entre -25°C et la température ambiante et dans un solvant aprotique apolaire, un dérivé de benzofuranne de formule générale : 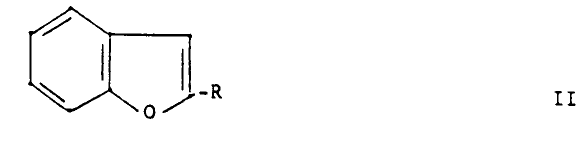 dans laquelle R a la même signification que précédemment, d'abord avec le phosgène ou le chlorure d'oxalyle, ensuite avec un dérivé phénolique de formule générale : 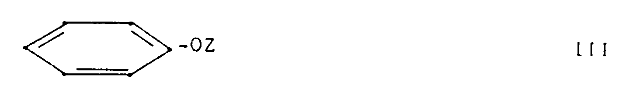 dans laquelle Z à la même signification que précédemment, pour obtenir un complexe que l'on hydrolyse pour former le composé désiré de benzoyl-3 benzofuranne.

2. Procédé selon la revendication 1, caractérisé en ce que les rapports molaires : dérivé de benzofuranne/dérivé phénolique/phosgène ou chlorure d'oxalyle/chlorure d'aluminium sont 1 / 1 / 2 à 4 / 1 à 1,5.

3. Procédé selon la revendication 2, caractérisé en ce que les rapports molaires sont 1 / 1 / 3 /1,5.

4. Procédé selon la revendication 1 pour la préparation d'un dérivé de benzoyl-3 benzofuranne de formule I dans laquelle Z représente l'hydrogène, caractérisé en ce que l'on fait réagir successivement in situ en présence de chlorure d'aluminium, comme catalyseur, à une température comprise entre -25°C et la température ambiante et dans un solvant aprotique apolaire, un dérivé de benzofuranne de formule II d'abord avec le phosgène, ensuite avec l'anisole pour obtenir un complexe que l'on déméthyle in situ à la température de reflux du milieu en présence de chlorure d'aluminium, puis on hydrolyse pour obtenir les dérivés de benzoyl-3 benzofuranne de formule I dans laquelle Z représente l'hydrogène.

5. Procédé selon la revendication 4, caractérisé en ce que la quantité de chlorure d'aluminium en tant que catalyseur est de 1 à 1,5 équivalent par équivalent de dérivé de benzofuranne de formule II et la quantité de chlorure d'aluminium nécessaire à la déméthylation est d'un équivalent par équivalent de dérivé de benzofuranne de formule II.

6. Procédé selon l'une des revendications 1 à 5, caractérisé en ce que le solvant aprotique apolaire est le tétrachlorure de carbone ou un halogénure d'alkyle en C₁-C₄.

7. Procédé selon l'une des revendications 1 à 6, caractérisé en ce que l'hydrolyse a lieu à une température comprise entre la température ambiante et 50°C.

8. Procédé selon la revendication 7, caractérisé en ce que l'hydrolyse a lieu à la température ambiante.

9. Procédé selon l'une des revendications 1 à 8, caractérisé en ce que R représente le radical éthyle.

10. Procédé selon l'une des revendications 1 à 8, caractérisé en ce que R représente le radical n-butyle.

## Claims

1. Process for preparing 3-benzoyl-benzofuran derivatives of general formula 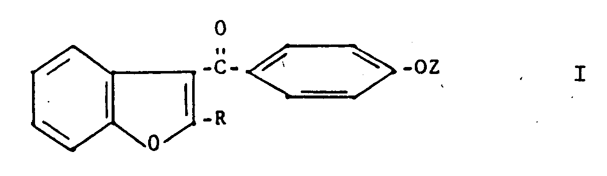 wherein
R is selected from a straight-chained or branched C₁₋₈-alkyl radical, a C₃₋₆-cycloalkyl radical or an unsubstituted phenyl or a phenyl substituted by one or more identical or different substituents selected from among the halogen atoms, C₁₋₄-alkyl groups, C₁₋₄-alkoxy groups and nitro,
Z denotes hydrogen or a methyl radical,
characterised in that a benzofuran derivative of general formula 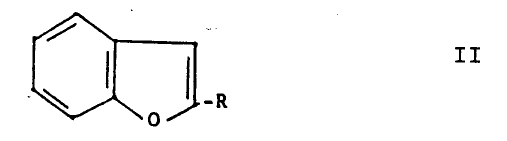 wherein R is as hereinbefore defined, is reacted successively in situ, in the presence of aluminium chloride as catalyst, at a temperature between -25°C and ambient temperature and in an apolar aprotic solvent, first with phosgene or oxalyl chloride, then with a phenol derivative of general formula 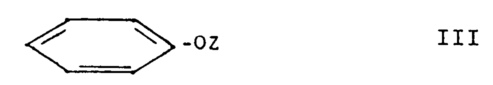 wherein Z is as hereinbefore defined, to obtain a complex which is hydrolysed to form the desired 3-benzoyl-benzofuran compound.

2. Process according to claim 1, characterised in that the molar ratios of the benzofuran derivatives/phenolic derivatives/phosgene or oxalyl chloride/aluminium chloride are 1:1:2-4:1-1.5.

3. Process according to claim 2, characterised in that the molar ratios are 1:1:3:1.5.

4. Process according to claim 1 for preparing a 3-benzoyl-benzofuran derivative of formula I wherein Z represents hydrogen, characterised in that a benzofuran derivative of formula II is reacted successively, in situ, in the presence of aluminium chloride as catalyst, at a temperature between -25°C and ambient temperature and in an apolar aprotic solvent, first with phosgene, then with anisole to obtain a complex which is demethylated in situ at the reflux temperature of the medium in the presence of aluminium chloride, then this complex is hydrolysed to obtain 3-benzoyl-benzofuran derivatives of formula I wherein Z denotes hydrogen.

5. Process according to claim 4, characterised in that the quantity of aluminium chloride as catalyst is 1 to 1.5 equivalents per equivalent of benzofuran derivative of formula II and the quantity of aluminium chloride required for the demethylation is one equivalent per equivalent of benzofuran derivative of formula II.

6. Process according to one of claims 1 to 5, characterised in that the apolar aprotic solvent is carbon tetrachloride or a C₁₋₄-alkyl halide.

7. Process according to one of claims 1 to 6, characterised in that the hydrolysis is carried out at a temperature between ambient temperature and 50°C.

8. Process according to claim 7, characterised in that the hydrolysis is carried out at ambient temperature.

9. Process according to one of claims 1 to 8, characterised in that R represents the ethyl radical.

10. Process according to one of claims 1 to 8, characterised in that R denotes the n-butyl radical.

## Patentansprüche

1. Verfahren zur Herstellung von 3-Benzoyl-benzofuran-Derivaten der allgemeinen Formel: 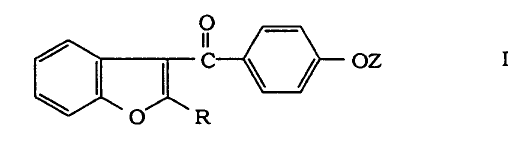 in der
R aus geradkettigen oder verzweigten C₁-C₈-Alkylgruppen, C₃-C₆-Cycloalkylgruppen und Phenylgruppen, die nichtsubstituiert oder durch einen oder mehrere identische oder verschiedenartige Substituenten ausgewählt aus Halogenatomen, C₁-C₄-Alkylgruppen, C₁-C₄-Alkoxygruppen und Nitrogruppen substituiert sein können, ausgewählt ist, und
Z aus Wasserstoff und Methylgruppen ausgewählt ist,
**dadurch gekennzeichnet**, daß man ein Benzofuranderivat der allgemeinen Formel: 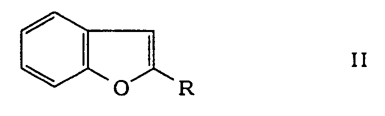 in der R die oben angegebenen Bedeutungen besitzt, in situ in Gegenwart von Aluminiumchlorid als Katalysator, bei einer Temperatur zwischen -25°C und Raumtemperatur und in einem apolaren aprotischen Lösungsmittel nacheinander zunächst mit Phosgen oder Oxalylchlorid und anschließend mit einem Phenolderivat der allgemeinen Formel: 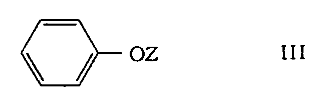 in der Z die oben angegebenen Bedeutungen besitzt, umsetzt zur Bildung eines Komplexes, den man zur Bildung der gewünschten 3-Benzoyl-benzofuran-Verbindung hydrolysiert.

2. Verfahren nach Anspruch 1, **dadurch gekennzeichnet**, daß das Benzofuranderivat/Phenolderivat/Phosgen oder Oxalylchlorid/Aluminiumchlorid-Molverhältnis 1/1/2 bis 4/1 bis 1,5 beträgt.

3. Verfahren nach Anspruch 2, **dadurch gekennzeichnet**, daß das Molverhältnis 1/1/3/1,5 beträgt.

4. Verfahren nach Anspruch 1 zur Herstellung eines 3-Benzoyl-benzofuran-Derivats der Formel I, in der Z Wasserstoff bedeutet, **dadurch gekennzeichnet**, daß man ein Benzofuranderivat der Formel II in situ in Gegenwart von Aluminiumchlorid als Katalysator bei einer Temperatur zwischen -25°C und der Raumtemperatur in einem apolaren aprotischen Lösungsmittel nacheinander zunächst mit Phosgen und anschließend mit Anisol umsetzt zur Bildung eines Komplexes, den man in situ bei der Rückflußtemperatur des Mediums und in Gegenwart von Aluminiumchlorid demethyliert und dann hydrolysiert zur Bildung der 3-Benzoyl-benzofuran-Derivate der Formel I, in der Z Wasserstoff bedeutet.

5. Verfahren nach Anspruch 4, **dadurch gekennzeichnet**, daß die Menge des als Katalysator verwendeten Aluminiumchlorids 1 bis 1,5 Äquivalente pro Äquivalent des Benzofuranderivats der Formel II beträgt und die Menge des für die Demethylierung notwendigen Aluminiumchlorids 1 Äquivalent pro Äquivalent des Benzofuranderivats der Formel II beträgt.

6. Verfahren nach einem der Ansprüche 1 bis 5, **dadurch gekennzeichnet**, daß das apolare aprotische Lösungsmittel Tetrachlorkohlenstoff oder ein C₁-C₄-Alkylhalogenid ist.

7. Verfahren nach einem der Ansprüche 1 bis 6, **dadurch gekennzeichnet**, daß die Hydrolyse bei einer Temperatur zwischen Raumtemperatur und 50°C durchgeführt wird.

8. Verfahren nach Anspruch 7, **dadurch gekennzeichnet**, daß die Hydrolyse bei Raumtemperatur durchgeführt wird.

9. Verfahren nach einem der Ansprüche 1 bis 8, **dadurch gekennzeichnet**, daß R eine Ethylgruppe darstellt.

10. Verfahren nach einem der Ansprüche 1 bis 8, **dadurch gekennzeichnet**, daß R die n-Butylgruppe bedeutet.
